# NOUVEAU FASCICULE DE BREVET EUROPEEN

(11) **EP 0 373 044 B2**
(45) Date de publication et mention de la décision concernant l'opposition: **03.09.2003**
(45) Mention de la délivrance du brevet: 19.10.1994
(21) Numéro de dépôt: 89403319.0
(22) Date de dépôt: 30.11.1989
(51) Int. Cl.: C12N 5/00, A61L 27/00, C07K 14/00, A61K 35/16

(54) **Support biologique pour cultures cellulaires constitué de protéines plasmatiques coagulées par la thrombine, son utilisation pour la culture des kératinocytes, leur récupération et leur transport à des fins d'utilisation thérapeutique**
Biologische Unterlage für Zellkulturen, bestehend aus durch Thrombin koagulierte Plasmaproteine, ihre Verwendung für die Keratinozytenkultur, deren Rückgewinnung und Transport für therapeutische Verwendungszwecke
Biological support for cell cultures constituted by plasma proteins coagulated by thrombin, its use in keratinocytes culture, their recuperation and transport for therapeutic use

(30) Priorité: 06.12.1988 FR 8815950
(43) Date de publication de la demande: 13.06.1990
(73) Titulaire: CENTRE REGIONAL DE TRANSFUSION SANGUINE DE LILLE, 59012 Lille (FR)
(72) Inventeur: Broly, Hervé, F-59116 Houplines (FR); Ronfard, Vincent, F-59130 Lambersart (FR)
(74) Mandataire: Lepeudry-Gautherat, Thérèse

(56) Documents cités:
- EP-A- 0 085 923
- EP-A- 0 143 648
- EP-A- 0 305 243
- EP-A- 0 305 243
- US-A- 4 442 655
- US-A- 4 452 893
- Redl et al. (1985). Die Medizinische Welt, Vol. 22. p. 3-10
- Porter et al. (1947), Proc. Soc. Exp. Biol. Vol. 65, p. 209-314
- Rupp (1988) Fibrinklebung in der Verbrennungschirurgie-Plastischen Chirurgie, Springer Verlag, p. 83-88
- Cormack (1987) Ham's Histology 9th Edition, p. 452.
- Green et al. (1979) PNAS, Vol. 76, No. 11. p. 5665-5668.
- Franke et al. (1986), dans "Neue Techniken in der Operativen Medizin" Hrsg. von M Reifferscheid Springer-Verlag, Berlin Heidelberg, p. 13-21.

## Description

La présente invention concerne l'utilisation pour la culture des kératinocytes et leur transport sous forme d'épidermes reconstitués d'un support biologique pour cultures cellulaires, constitué du mélange coagulé d'un concentré de protéines plasmatiques et de thrombine et l'utilisation desdits épidermes reconstitués à des fins thérapeutiques.

La reconstruction en laboratoire d'une peau vivante semblable à la peau humaine à partir de quelques cellules d'une biopsie ou d'une peau simplifiée assurant les fonctions physiologiques de la peau normale fait l'objet de nombreuses études dans le but de remplacer la peau lésée par une affection grave (génétique ...) ou détruite par d'importantes brûlures.

La peau est un organe complexe composé de trois tissus juxtaposés : l'épiderme, constitué à 85 % de kératinocytes qui élaborent la couche cornée imperméable qui isole le corps du milieu extérieur; le derme qui comporte des cellules, dont les fibrocytes, séparés par un tissu conjonctif constitué surtout de collagène ; le derme repose sur l'hypoderme qui comprend les cellules spécialisées dans le stockage des graisses. La reconstruction artificielle d'un organe aussi complexe pose donc de nombreux problèmes.

Le premier tissu qui a été partiellement reconstruit in vitro est le derme, par l'équipe de Bell (Bell et al. Proc. Natl. Acad. Sci. 76 - 1979 - 1274). A partir de biopsies de peau, les fibroblastes ont pu être mis en culture, d'abord en monocouches puis, après quelques passages, en dispersant ces cellules dans du milieu de culture contenant du collagène (extrait de tendons de queues de rats), celui-ci constituant un gel et permettant une culture tridimensionnelle. On voit dans cette culture les fibroblastes interagir avec la matrice du collagène, l'organiser et la contracter comme dans un derme normal. Ce tissu reconstruit in vitro est connu sous le nom de "derme équivalent". Après quelques semaines de culture, les qualités mécaniques du derme équivalent permettent de l'utiliser pour greffer un malade ou un blessé. Il ne semble pas être rejeté par le receveur. Toutefois ce derme équivalent n'est qu'un pansement provisoire : il ne peut pas restaurer la fonction de barrière cutanée.

D'autre part, l'équipe de Green (H. Green et al. Proc. Natl. Adac. Sci 76, 1979, 5665) a mis au point une méthode et un milieu de culture permettant de cultiver de façon prolongée des kératinocytes. Cette méthode comprend l'inoculation des kératinocytes dispersés à la trypsine sur une monocouche préétablie de fibroblastes, en particulier des cellules 3T3, létalement irradiées et qui servent de couche nourricière et de matrice. Le feuillet épidermique se développe très rapidement pour former un tissu de 3 à 5 cellules d'épaisseur;il pourra être greffé sur un patient et continuer à se différencier in situ. Des grands brûlés ont déjà pu être sauvés par cette technique ( G. Gallico et al. New England J. Med. 311, 1984, 448).

Par la technique de Green on peut obtenir, à partir d'une biopsie de deux centimètres carrés, un épiderme de un mètre carré après trois semaines.

La récupération du tissu reconstitué pour en faire un greffon pose encore des problèmes techniques. En effet, il faut décoller les cellules de la boîte de culture, par un traitement enzymatique, sans les dissocier entre elles ; au cours de cette opération on observe toujours une rétraction du tapis cellulaire et donc une perte d'un certain pourcentage de la superficie du greffon. Le tissu reconstitué étant détaché, il faut l'agrafer sur un support permettant son transport et sa greffe sur le patient. On utilise généralement un pansement de gaze vaselinée. Cet ensemble de manipulations est délicat et prend beaucoup de temps.

Il serait donc très bénéfique de disposer de nouveaux supports biologiques, résorbables au cours du temps par le patient greffé et simplifiant la manipulation des cellules. De plus, pour en garantir la disponibilité, ces supports ou leurs constituants devraient être susceptibles d'être préparés et conditionnés selon des procédés industriels.

La Demanderesse a ainsi mis au point un support biologique pour cultures cellulaires, constitué du mélange d'un concentré de protéines plasmatiques coagulables par la thrombine et de la quantité de thrombine calcique nécessaire pour déclencher la coagulation.

La coagulation des protéines du plasma en présence de thrombine, est due principalement à la formation d'un réseau de fibrine polymérisée, qui mime la formation du caillot sanguin. Pour constituer un support adapté à la culture des cellules, la coagulation est réalisée dans des conditions favorisant la formation d'un film et plus particulièrement dans des botes de Petri ou dans tout flacon adapté à la culture de cellules.

Le concentré de protéines plasmatiques a déjà été décrit par la Demanderesse dans la demande de brevet européen 88.401 961.3 : il est obtenu par précipitation de plasma frais, par deux traitements successifs à l'éthanol à 10 %, à 4°C. Le concentré comprend plus de 90 % de fibrinogène, et, par gramme de protéines, au moins 0,1 Ul de Facteur XIII et de 0,03 à 0,1 gramme de fibronectine. Le concentré est conditionné et lyophilisé pour être conservé jusqu'à son utilisation.

La présente invention concerne donc l'utilisation du film, compatible avec la multiplication des cellules de type kératinocytes humaines, et obtenu :
- par le conditionnement séparé et la lyophilisation de chacun des deux composants :
   - a) un concentré de protéines coagulables par la thrombine, obtenu par traitement à l'éthanol de plasma humain, et contenant des proportions équilibrées de fibrinogène, et de préférence plus de 90 %, de facteur XIII, et de préférence au moins 0/1 UI/g, et de fibronectine, et de préférence de 0,03 à 0,1 g/g,
   - b) la quantité de thrombine calcique nécessaire pour déclencher la coagulation,
- la simple remise en solution aqueuse des deux composants a) et b),
- leur mélange sur une boîte de type boîte de Petri
- et par ajustement de la pression osmotique dudit film à une pression physiologique comprise entre 260 et 340 mosM, de préférence avec du milieu de culture pour cellules,
pour constituer, par culture en employant ledit film comme support biologique de kératinocytes humains, un tissu de remplacement directement récupérable, transportable et applicable comme greffon.

Au moment de son utilisation, le concentré de protéines coagulables par la thrombine est redissous dans une solution aqueuse saline ou dans une solution contenant un inhibiteur polyvalent des protéases, préférentiellement l'aprotinine, à une concentration de 3000 UlK/ml.

Pour déclencher le processus de coagulation et donc la formation du film servant de support pour les cellules, on ajoute de la thrombine avec ou sans calcium. Le processus comprend la transformation du fibrinogène en fibrine sous l'action de la thrombine et la polymérisation de la fibrine monomère avec la fibronectine sous l'action du Facteur XIII activé par les ions Ca⁺⁺.

Pour constituer le support selon l'invention, particulièrement favorable aux cultures cellulaires, on ajuste de préférence la concentration de thrombine à environ 10 Ul/ml (concentration beaucoup plus faible que celle qu'on utilise quand'la consistance recherchée est différente, comme dans le cas des colles biologiques - brevet 88.401 961.3, cité plus haut - ).

Selon différents modes de réalisation de l'invention, on peut incorporer dans le support divers additifs particulièrement destinés à favoriser la multiplication cellulaire in vitro ou in situ et ainsi favoriser la cicatrisation de la plaie après la greffe.

Le support peut donc contenir un additif favorisant la multiplication des cellules comme un facteur de croissance et plus particulièrement l'EGF ("epidermal growth factor").

On peut aussi incorporer un agent cicatrisant et/ou un antibiotique.

Le support selon l'invention est particulièrement favorable à la culture des kératinocytes humains. Ces cellules peuvent être soit des cultures primaires dérivées de biopsies de peau d'un patient et ayant subi entre 1 et 4 passages en dilution 1/15 à 1/20, soit des cellules conservées sous forme de banque dans l'azote liquide.

Ces kératinocytes établis en tapis confluent sont trypsinés et remis en suspension dans du milieu de culture approprié au moment de leur ensemencement sur le support selon l'invention.

L'utilisation du support biologique selon l'invention peut être adaptée de trois manières différentes.

Selon un premier mode d'utilisation, le support biologique est préparé sous forme de film, par le mélange de ses deux constituants sur une boîte de culture; une suspension de kératinocytes est ensemencée sur ce film, dans du milieu de culture approprié.Quand la culture de kératinocytes est devenue confluente ou semi-confluente;elle constitue un tissu de remplacement directement récupérable comme greffon, qu'on peut détacher à l'aide de pinces et transporter de la boîte sur le patient auquel il est appliqué tel quel, sans nécessiter de support transitoire tel que la gaze. Ceci permet un important gain de temps opératoiré et une récupération de 100 % du tissu en culture.

Selon un autre mode d'utilisation du support selon l'invention, les deux constituants du support sont mélangés avec la suspension de kératinocytes de manière à intégrer les cellules dans le film qui se formera ultérieurement. Selon ce mode d'utilisation, le mélange des deux constituants avec la suspension de cellules peut être réalisé sur une boite de culture et être ensuite uitlisé comme greffon, comme dans le mode d'utilisation précédent; il peut aussi être directement réalisé sur la plaie du patient, préparée pour accueillir un greffon,notamment en pulvérisation du support biologique et des cellules à l'aide d'un gaz vecteur (azote) sous une pression de 2 à 2,5 bars.

Selon un autre mode d'utilisation du support selon l'invention, le mélange de ses deux constituants est réalisé sur un tapis cellulaire de kératinocytes pré-établi dans une boîte de culture, de telle manière que les cellules soient enrobées dans le film formé et puissent ainsi être détachées et transportées pour être appliquées comme greffon.

Les exemples suivants illustrent l'invention sans toutefois en limiter la portée.

### Exemple I - Préparation du support biologique pour cultures cellulaires.

On prépare un support biologique pour cultures cellulaires en mélangeant un concentré de protéines plasmatiques coagulables et la quantité de thrombine calcique nécessaire pour déclencher la coagulation.

### A. Préparation du concentré de protéines plasmatiques

La préparation du concentré de protéines a déjà été décrite par la Demanderesse dans la demande de brevet européen 88.401 961.3. En résumé, on utilise du plasma humain non cryoprécipité; on le soumet à deux précipitations successives à l'éthanol à 10 %, à pH 7,2 et à 4°C. Entre les deux précipitations, le produit subit un traitement d'inactivation virale. Le précipité, séparé du sumageant par centrifugation, est lavé à l'éthanol à 4°C et recentrifugé. Le précipité est remis en suspension dans du tampon Tris/citrate, ajusté à une concentration en protéines de environ 35 g/l et additionné de lysine à une concentration finale de 0,1 à 0,2 g par gramme de protéines. Après diafiltration pour éliminer l'alcool et le citrate et pour ajuster la force ionique, le concentré est conditionné en flacons et lyophilisé.

Ce concentré de protéines renferme, pour un gramme de protéines, au moins 0,9 g de fibrinogène, de 0,03 à 0,06 g de fibronectine et de 0,15 à 0,30 Ul de Facteur XIII.

### B. Préparation du support pour cultures cellulaires

Le concentré de protéines décrit plus haut est remis en suspension dans une solution aqueuse,avec ou sans aprotinine à 3000 UIK/ml (unités d'inhibiteur de kallicréine/ml)

On mélange cette solution avec un volume égal de thrombine calcique à 10 Ul/ml. Pour une boîte de Pétri de 10 cm de diamètre on utilise 2 ml de suspension de protéines et 2 ml de thrombine, ces deux solutions étant injectées simultanément par deux seringues reliées par un raccord mélangeur. On agite la boîte de Petri de manière à obtenir une répartition uniforme puis on laisse reposer pendant 15 à 20 minutes. La préparation forme un film qui recouvre la boîte.

Les boîtes de culture sont de qualité "non traitéepour culture cellulaire" ce qui permet au support de ne pas adhérer de façon permanente ce qui facilite sa récupération ultérieure.

Ce film est ensuite recouvert de milieu de culture pour cellules. Ce milieu est renouvelé plusieurs fois jusqu'à ce que la pression osmotique du film soit stabilisée dans une gamme compatible avec la physiologie des cellules, c'est-à-dire entre 260 et 340 m o s M(milliosmoles).

Alternativement, on peut dialyser le concentré de protéines reconstitué avant de le mélanger à la thrombine.

### Exemple 2. - Mise en culture de kératinocytes sur le support biologique.

On utilise des cultures primaires de kératinocytes réalisées selon la technique classique de Green à partir de biopsie(s) de peau d'un patient (ou de peau d'un embryon pour constituer des banques de cellules foetales). Ces cultures primaires peuvent subir 4 à 5 passages en dilution 1/10.

Un tapis de kératinocytes confluents est trypsiné, remis en suspension dans du milieu de culture et ensemencé en dilution 1/10 sur une boîte de Petri recouverte d'un film du support biologique décrit dans l'exemple 1.

Après quelques heures, les cellules adhèrent au support; elles s'y multiplient ensuite normalement jusqu'à former un fragment d'épiderme confluent et d'une épaisseur de 3 ou 4 couches de cellules.

Ce fragment d'épiderme reconstitué, adhérent au support, peut être détaché de la boîte de culture à l'aide de pinces et appliqué tel quel sur une plaie préparée pour accueillir un greffon.

Grâce à l'adhérence des cellules au support, il n'est pas nécessaire d'agrafer l'épiderme reconstitué sur un autre support tel que la gaze vaselinée qui doit être utilisée avec les autres types de cultures. Ceci permet un important gain de temps opératoire : on arrive à manipuler 40 boîtes à l'heure au lieu de 4 par les techniques classiques.

De plus, ce support résiste bien aux manipulations et ne se rétracte pas au moment où on le détache, ce qui permet de récupérer 100 % de la superficie du tapis cellulaire en culture.

### Exemple 3 - Récupération d'un tapis cellulaire préétabli à l'aide du support biologique.

Des kératinocytes sont mis en culture selon la méthode classique de Green, dans une boîte de Petri couverte d'une couche de fibroblastes létalement irradiés.

Quand le tapis de kératinocytes est confluent et constitué de qualques couches de cellules, le milieu de culture est éliminé, on ajoute une solution d'EDTA durant 1 heure 30, puis on effectue deux lavages en PBS. On coule ensuite le support biologique directement sur le tapis de cellules, selon la méthode décrite dans l'exemple 2.

Quand le film est formé sur les cellules, il peut être détaché avec des pinces et utilisé comme greffon, comme dans l'exemple précédent.

### Exemple 4 - Incorporation des cellules dans le support biologique.

On prépare une seringue de solution de protéines et une seringue de thrombine contenant les kératinocytes en suspension. Ces kératinocytes peuvent provenir d'une culture fraîche, trypsinée, ou d'une banque de cellules conservées à l'azote liquide.

Les deux seringues sont reliées à l'aide d'un raccord mélangeur et on pulvérise le support contenant les cellules sur la boîte de Pétri (ou sur la plaie à greffer) ; les cellules se trouvent ainsi prises dans le film pendant sa coagulation.Cette pulvérisation peut se faire à l'aide d'un gaz vecteur (l'azote à une pression de 2 à 2,5 bars).

Cette pulvérisation ne dénature pas les cellules et on observe la reformation d'un tapis cellulaire, en culture in vitro. Les cellules devraient donc se multiplier normalement ou quasiment normalement quand le mélange est pulvérisé, en couche très fine, directement sur une plaie.

## Revendications

1. Utilisation du film, compatible avec la multiplication des cellules de type kératinocytes humaines, et obtenu :
- par le conditionnement séparé et la lyophilisation de chacun des deux composants :
- a) un concentré de protéines coagulables par la thrombine, obtenu par traitement à l'éthanol de plasma humain, et contenant des proportions équilibrées de fibrinogène, et de préférence plus de 90 %, de facteur XIII, et de préférence au moins O,I UI/g, et de fibronectine, et de préférence de 0,03 à 0,1 g/g,
- b) la quantité de thrombine calcique nécessaire pour déclencher la coagulation,
- la simple remise en solution aqueuse des deux composants a) et b),
- leur mélange sur une boîte de type boîte de Petri
- et par ajustement de la pression osmotique dudit film à une pression physiologique comprise entre 260 et 340 mosM, de préférence avec du milieu de culture pour cellules,
pour constituer, par culture en employant ledit film comme support biologique de kératinocytes humains, un tissu de remplacement directement récupérable, transportable et applicable comme greffon.

2. Utilisation selon la revendication 1, **caractérisée en ce que** la thrombine calcique est mélangée avec le concentré de protéines remis en solution, à une dose de environ 10 UI/ml.

3. Utilisation selon la revendication 1 ou 2, **caractérisée en ce que** ledit film contient, comme additif, une substance favorisant la multiplication cellulaire.

4. Utilisation selon l'une quelconque des revendications 1 à 3, **caractérisée en ce que** ledit film contient, comme additif, un antibiotique.

5. Utilisation selon l'une quelconque des revendications 1 à 4, **caractérisée en ce que** ledit film contient, comme additif, une subtance favorisant la cicatrisation des plaies.

6. Utilisation selon l'une quelconque des revendications 1 à 5, **caractérisée en ce que** le mélange des deux constituants du support biologique est réalisé de manière à constituer un film uniforme dans une boîte de culture et que les kératinocytes en suspension dans du milieu de culture sont ensemencés sur ce film.

7. Utilisation selon l'une quelconque des revendications 1 à 5, **caractérisée en ce que** les deux constituants du support biologique sont mélangés avec une suspension de kératinocytes de manière à intégrer les cellules dans le film formé ultérieurement.

8. Utilisation selon la revendication 6 ou 7, **caractérisée en ce que** la suspension de kératinocytes est réalisée après dispersion d'un tapis cellulaire préétabli, frais.

9. Utilisation selon la revendication 6 ou 7, **caractérisée en ce que** la suspension de kératinocytes est réalisée à partir d'une banque de cellules conservées dans l'azote liquide.

10. Utilisation selon l'une quelconque des revendications 1 à 9, pour la récupération et le transport d'une culture préétablie de kératinocytes humains.

11. Utilisation selon la revendication 10, **caractérisée en ce que** le mélange des deux constituants du support biologique est réalisé sur le tapis cellulaire préétabli dans une boîte de culture.

## Claims

1. Use of a film, compatible with the multiplication of human keratinocyte-type cells and obtained:
- by separately packaging and freeze-drying each of the following two components:
a) a concentrate of proteins that can be coagulated by thrombin, which concentrate is obtained by treating human plasma with ethanol and contains balanced proportions of fibrinogen, preferably more than 90%, of factor XIII, preferably at least 0.1 IU/g, and of fibronectin, preferably from 0.03 to 0.1 g/g,
b) the quantity of calcium thrombin necessary to activate coagulation,
- simply redissolving in aqueous solution the two components a) and b),
- mixing them in a dish of the Petri dish type,
- and adjusting the osmotic pressure of the said film to a physiological pressure of from 260 to 340 mosM, preferably using cell culture medium,
in order to form, by culturing with the use of the said film as a biological support for human keratinocytes, tissue that is directly recoverable, transportable and applicable as a graft

2. Use according to claim 1, **characterised in that** the calcium thrombin is mixed with the redissolved protein concentrate in an amount of approximately 10 IU/ml.

3. Use according to claim 1 or 2, **characterised in that** the said film contains, as an additive, a substance that promotes cell multiplication.

4. Use according to any one of claims 1 to 3, **characterised in that** the said film contains, as an additive, an antiobiotic.

5. Use according to any one of claims 1 to 4, **characterised in that** the said film contains, as an additive, a substance that promotes the healing of wounds.

6. Use according to any one of claims 1 to 5, **characterised in that** the two constituents of the biological support are mixed in such a manner as to form a uniform film in a culture dish and that the keratinocytes suspended in the culture medium are seeded onto that film.

7. Use according to any one of claims 1 to 5, **characterised in that** the two constituents of the biological support are mixed with a suspension of keratinocytes in such a manner as to integrate the cells into the film subsequently formed.

8. Use according to claim 6 or 7, **characterised in that** the suspension of keratinocytes is prepared after dispersion of a fresh, pre-established cell layer.

9. Use according to claim 6 or 7, charactersed in that the suspension of keratinocytes is prepared from a bank of cells preserved in liquid nitrogen.

10. Use according to any one of claims 1 to 9 in the recovery and transport of a pre-established culture of human keratinocytes.

11. Use according to claim 10, **characterised in that** the two constituents of the biological support are mixed on the pre-established cell layer in a culture dish.

## Patentansprüche

1. Verwendung des Films, kompatibel mit der Vermehrung von Zellen vom Typ menschlicher Keratinocyten und erhalten:
- durch die getrennte Konditionierung und die Lyophilisation jedes der beiden Bestandteile:
- a) einem Konzentrat von durch Thrombin koagulierbaren Proteinen, das durch Behandeln von menschlichem Plasma mit Ethanol erhalten wird und ausgeglichene Anteile von Fibrinogen, und vorzugsweise mehr als 90 %, von Faktor XIII, und vorzugsweise mindestens 0,1 IE/g, und von Fibronectin, und vorzugsweise 0,03 bis 0,1 g/g, enthält,
- b) der Menge an Calcium-Thrombin, die erforderlich ist, um die Koagulation auszulösen,
- das einfache Wiederauflösen der beiden Bestandteile a) und b) in wässriger Lösung,
- ihre Vermischung in einem Gefäß von der Art einer Petrischale,
- und durch Einstellen des osmotischen Drucks dieses Films auf einen physiologischen Druck zwischen 260 und 340 mosM, vorzugsweise mit Zellkulturmedium,
um - indem der genannte Film als biologische Unterlage von menschlichen Keratinocyten benutzt wird -, durch Kultur ein Ersatzgewebe zu bilden, das direkt gewonnen bzw. entnommen, transportiert und als Transplantat verwendet werden kann.

2. Verwendung nach Anspruch 1, **dadurch gekennzeichnet, dass** das Calcium-Thrombin mit dem wieder in Lösung gebrachten Proteinkonzentrat in einer Dosis von ungefähr 10 IE/ml vermischt wird.

3. Verwendung nach Anspruch 1 oder 2, **dadurch gekennzeichnet, dass** der genannte Film als Additiv eine Substanz enthält, welche die Zellvermehrung begünstigt.

4. Verwendung nach einem der Ansprüche 1 bis 3, **dadurch gekennzeichnet, dass** der genannte Film als Additiv ein Antibiotikum enthält.

5. Verwendung nach einem der Ansprüche 1 bis 4, **dadurch gekennzeichnet, dass** der genannte Film als Additiv eine Substanz enthält, welche die Wundheilung begünstigt.

6. Verwendung nach einem der Ansprüche 1 bis 5, **dadurch gekennzeichnet, dass** das Gemisch aus den beiden Bestandteilen der biologischen Unterlage derart hergestellt wird, dass es einen einheitlichen Film in einem Kulturgefäß bildet und dass die Keratinocyten in Suspension in Kulturmedium auf diesen Film ausgesät werden.

7. Verwendung nach einem der Ansprüche 1 bis 5, **dadurch gekennzeichnet, dass** die beiden Bestandteile der biologischen Unterlage mit einer Keratinocytensuspension derart vermischt werden, dass die Zellen in den später gebildeten Film integriert sind.

8. Verwendung nach Anspruch 6 oder 7, **dadurch gekennzeichnet, dass** die Keratinocytensuspension nach der Dispergierung eines zuvor bereits bestehenden frischen Zellrasens hergestellt wird,

9. Verwendung nach Anspruch 6 oder 7, **dadurch gekennzeichnet, dass** die Keratinocytensuspension aus einer in flüssigem Stickstoff konservierten Zellbank hergestellt wird.

10. Verwendung nach einem der Ansprüche 1 bis 9 zur Gewinnung bzw. Entnahme und zum Transport einer zuvor bereits bestehenden Kultur von menschlichen fötalen oder adulten Keratinocyten.

11. Verwendung nach Anspruch 10, **dadurch gekennzeichnet, dass** das Gemisch der beiden Bestandteile der biologischen Unterlage auf dem zuvor bereits bestehenden Zellrasen in einem Kulturgefäß hergestellt wird.
